(19) Europäisches Patentamt
European Patent Office
Office européen des brevets

(11) EP 1 786 486 B1

# (12) EUROPEAN PATENT SPECIFICATION

(45) Date of publication and mention of the grant of the patent:
**18.05.2011 Bulletin 2011/20**

(21) Application number: **05781070.7**

(22) Date of filing: **08.09.2005**

(51) Int Cl.:
***A61L 27/52*** *(2006.01)* ***A61L 27/50*** *(2006.01)*

(86) International application number:
**PCT/NL2005/000650**

(87) International publication number:
**WO 2006/028370 (16.03.2006 Gazette 2006/11)**

## (54) RADIOPAQUE PROSTHETIC INTERVERTEBRAL DISC NUCLEUS

RÖNTGENDICHTE BANDSCHEIBENKERNPROTHESE

PROTHESE DE NOYAU DE DISQUE INTERVERTEBRAL RADIO-OPAQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.09.2004 EP 04077514**

(43) Date of publication of application:
**23.05.2007 Bulletin 2007/21**

(73) Proprietor: **Stichting Dutch Polymer Institute**
**5612 AB Eindhoven (NL)**

(72) Inventors:
- **VAN HOOY-CORSTJENS, Catharina, Sibilla, Josephine**
  **NL-6116 BX Roosteren (NL)**
- **KOOLE, Levinus Hendrik**
  **NL-6271 EC Gulpen (NL)**
- **BOELEN, Erik, Johannes, Herman**
  **NL-6225 ED Maastricht (NL)**

(74) Representative: **Renkema, Jaap et al**
**Ipecunia B.V.**
**P.O. Box 593**
**6160 AN Geleen (NL)**

(56) References cited:
**WO-A-94/23671**
**WO-A-96/05872**
**US-A1- 2004 054 413**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 786 486 B1

## Description

This invention relates to a prosthetic nucleus for an intervertebral disc. More specifically, it relates to an artificial disc nucleus made of a hydrogel material.

The intervertebral disc (IVD) in a human body, or more generic in vertebrates, is a complex joint both anatomically as functionally. It is composed of three component structures: the nucleus pulposus (the nucleus), the annulus fibrosus (the annulus) and the vertebral end plates. The biochemical composition and anatomical arrangements within these component structures are related to the biomechanical function of the disc.

Degeneration or injury of the IVD is the most common cause of back pain. Back pain is the largest cause of lost workdays and activity limitation in western countries amongst people younger than 45 years.

In case conservative treatment as painkillers and physiotherapy do not suffice, surgical intervention (such as discectomy and fusion) is an option to alleviate the pain. However, these methods have some disadvantages. Discectomy usually is not desirable from a biomechanical point of view, since this causes increased compression load onto the annulus ring. This narrows the disc spaces in long term consequently increasing the load on the facet joints. Fusion restricts the motion of the fused segment(s), thereby restricting the mobility in the spine of the patient and increasing the risk of degeneration of the adjoining vertebral discs, since they have to make up for the loss in motion in the fused ones.

Another option is to use a prosthetic joint device, which is not only able to replace the injured or degenerated intervertebral disc, but also can mimic the physiological and the biomechanical function of the disc to be replaced and prevent further degeneration of the surrounding tissue. Such replacement is however quite complex and it is it hard to match nature's design.

In some cases, the annulus of the IVD is degenerated or ruptured in such a way that herniation of the nucleus pulposus occurs. Then, adequate treatment may be to remove the herniated nucleus and replace it with a prosthetic nucleus, followed by mending of the annulus defect. This treatment can for example be suitable in case of a traumatic rupture of the annulus or in case of early-stage disc degeneration.

Replacement of only the nucleus has several advantages over the interventions as mentioned above as it is less invasive and the remaining tissues, i.e, the annulus and the endplates, are preserved and so are their functions.

WO94/23671 discloses a prosthetic nucleus for a vertebral disc made of a hydrogel material. A disadvantage of this prosthetic nucleus is that it is not possible to visualize the implanted prosthetic nucleus during and after surgery. The success of a nucleus replacement is largely dependent on the correct positioning of the implant. To assess adequately the location of the implant during and after surgery, it is convenient that it can be monitored real-time using X-ray fluoroscopy. That way the surgeon is able to perfectly position the implant in the nucleus cavity. Also, in case of complications arising at a later time, the implant can be easily traced.

A prosthetic nucleus comprising radiopaque material used therein as a marker is disclosed in US 2004-0054413A1. The radiopaque marker is a metal (such as gold, tungsten, titanium, tantalum or platinum) and is located within the hydrogel, without being bound thereto.

A disadvantage of the use of such radiopaque marker is that only the marker is visible, not the prosthesis itself. In case of layers of radiopaque marker, the circumference of the prosthesis is not visible, thereby not giving sufficient information of its exact whereabouts. In case the radiopaque marker is dispersed in powder form, risk of diffusion out of the hydrogel is increasing, thereby also not enabling to locate the exact position of the prosthesis. Another disadvantage of metallic radiopaque markers is that they can cause artifacts in nuclear magnetic resonance imaging (MRI) disrupting the image, especially at high field strengths.

It is the object of the present invention to provide a prosthetic nucleus that can be seen integrally, whilst having homogeneity of the material.

This is surprisingly achieved by the use of a hydrogel based on a copolymer comprising:

(a) at least one monomer comprising covalently bound iodine or bromine, and;
(b) at least one hydrophilic monomer,

wherein the hydrogel, when fully hydrated, has an elastic modulus between 0.01-100 MPa at 37°C.
Copolymers comprising at least

(a) a monomer comprising covalently bound iodine or bromine
(b) at least one hydrophilic monomer

are known from for example WO96/05872. WO96/05872 however, discloses radiopaque polymers comprising at least one covalently bound iodine or bromine group which are not forming a hydrogel. The polymers disclosed comprise a too high amount of the hydrophobic monomer MMA, thereby making the formation of a hydrogel impossible. The amount of hydrophilic monomers in the copolymers disclosed in WO96/05872 does not exceed 20 mol%.

Furthermore, the elastic modulus of these types of materials is in the range of GPa's, whilst the materials according to the present invention are at least a factor 10 lower. The polymers as disclosed in WO96/05872 are suitable as biomedical construction material or as radiopacifier for bone cement or for dental filling, but not for the replacement of the nucleus pulposus.

The hydrogel according to the invention is intrinsically radiopaque, as a result of which the contours of the implant can be made visible with X-ray. Additionally, it has been found that the radiopacity of the hydrogel is stable in time. Furthermore, no leakage of material into the body takes place.

A copolymer is a material created by polymerizing a mixture of at least two monomers. Suitable combinations can for example exist of two monomers, three monomers (a so-called terpolymer), four monomers, etcetera.

The term hydrogel is here and hereafter used for a three-dimensional, hydrophilic, polymeric network capable of imbibing water or biological fluids. The networks are insoluble due to the presence of chemical or physical crosslinks, thus referring to such a polymeric network is regardless whether it has already imbibed fluids or not, unless explicitly stated otherwise (see also Peppas NA et al, Hydrogels in pharmaceutical formulations, Eur J Pharm Biopharm 2000 (50) p27-46). A hydrogel is sufficiently hydrated when the equilibrium water content (EWC) is reached. The EWC is reached once the mass of a wetted sample doesn't change significantly (± 2%) within a week, measured at 20°C. The EWC is calculated using the following equation:

$$EWC = 100\% \times \frac{m_s - m_d}{m_s}$$

wherein $m_s$ is the mass of the hydrated sample in equilibrium state and $m_d$ is the mass of the dry sample.

Any monomer comprising a covalently bound iodine or bromine is suitable for use in the hydrogel according to the invention. Suitable monomers are vinylic, acrylic and methacrylic monomers. Monomers of this type include, but are not limited to the group of structures represented in formula 1.

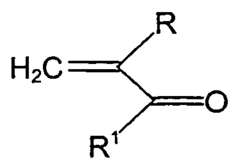

(I)

wherein R = H or an alkyl group having 1 or 2 C-atoms
and $R^1$ = I, Br or

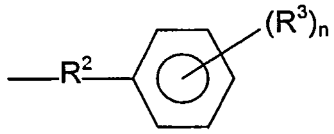

(II)

wherein $R^2$ = O, NH, O-$[CH_2$-$CH_2$-O]p-C(O)-, O-$[CH_2]_m$-O-C(O)-, O-$[CH_2]_P$-, NH-$[CH_2$-$CH_2$-O]p-C(O)-, NH-$[CH_2]_m$-O-C(O)- or NH-$[CH_2]_p$-wherein m > 1 and p ≥ 1, $R^3$ = I or Br and
nis1,2or3.

Methods of making such compounds are for example disclosed in WO96/05872. Preferably m or p are below 10. Preferably m is 2. Preferably p is 1 or 2. Most preferably m is 2 and p is 1 or 2.
$R^3$ can be located at all possible positions, being ortho, meta, and para. In case n=1, $R^3$ is preferably located at position 2 or 4. Most preferably at position 4. In case n=2, $R^3$ can be located at position 2 and 4 (ortho and para respectively) or position 3 and 5 (meta). In case n=3, $R^3$ is preferably located at positions 2,3 and 5.

Examples of suitable monomers comprising covalently bound iodine or bromine (a) are 2-[2'-iodobenzoyl]-oxo-ethylmethacrylate, 2-(4'-iodobenzoyl)-oxoethyl methacrylate (4IEMA) or 2-[2', 3', 5'-triiodobenzoyl]-oxo-ethyl methacrylate.

Preferably a monomer comprising covalently bound iodine is used. In a preferred embodiment 4IEMA is used

since this crystalline material can be easily prepared in bulk-quantities in pure form via radical polymerization. In another preferred embodiment, 2-[2', 3', 5'-triiodobenzoyl]-oxo-ethyl methacrylate is used, which is useful to introduce a high level of X-ray contrast in the copolymer, since during polymerization three iodine atoms are introduced per monomer.

Hydrophilic monomer is hereafter defined as any monomer having a strong affinity for water, tending to dissolve in, mix with, or be wetted by water. Examples of suitable hydrophilic monomers (b) are N-vinyl-2-pyrrolidinone (NVP), 2-hydroxy ethyl methacrylate (HEMA), methacrylic acid (MAA), polyethylene glycol methacrylate (PEG-MA), vinyl acetate as a precursor for vinyl alcohol (VA) or derivatives thereof. For the invention it is important that at least one hydrophilic monomer (b) is used in the copolymer, but also mixtures of hydrophilic monomers can be used. Preferably the hydrophilic monomer (b) is HEMA and/or NVP.

The amount of hydrophilic monomer (b) used to create the copolymer in the hydrogel according to the invention has to suffice to form a hydrogel. The characteristics of the hydrated hydrogel with respect to the equilibrium water content, swelling ratio and time to reach the equilibrium water content differs per hydrophilic monomer. In other words, in case of the same amount of hydrophilic monomer, these characteristics can differ substantially for different hydrophilic monomers. The person skilled in the art can however easily determine the desired amount of hydrophilic monomer in the hydrogel in order to obtain the right mechanical properties.

Generally, a hydrogel according to the invention can imbibe water up to an EWC of at least 10 wt.% . Preferably, the EWC of the hydrogel is at least 15 wt.%. Even more preferably the EWC of the hydrogel is at least 20 wt.% and most preferably at least 30 wt.%.

In order to give the hydrogel the right mechanical properties the hydrogel generally has a maximal EWC of 95 wt.% .

More preferably, the hydrogel has an EWC between 20 and 90 wt.%.

In another embodiment of the invention the copolymer also comprises a hydrophobic monomer (c). A hydrophobic monomer is hereafter defined as any monomer lacking affinity for water, tending to repel and not absorb water, tending not to dissolve in or mix with or be wetted by water. Examples of such monomers (c) are: methylmethacrylate (MMA), butylmethylacrylate (BMA), vinylacetate as precursor for polyvinyl acetate (VAp).

The suitable monomers in the copolymer of the present invention can have one or more reactive groups. In one embodiment of the invention at least one monomer is used having two or more reactive groups. In this embodiment a polymer network can be formed. Examples of monomers having two reactive groups, suitable for the present invention are dimethacrylates, divinylbenzene, or allylmethacrylate.

The fully hydrated hydrogel according to the invention preferably has an elastic modulus (Young's Modulus) of 0.01 -100 MPa at 37°C. Preferably, the elastic modulus of the hydrogel is at least 0.1 MPa, more preferably at least 0.2 MPa, and most preferably at least 0.3 MPa. In order to give the hydrogel the right mechanical properties the hydrogel generally has maximum elastic modulus of 100 MPa. Preferably, the elastic modulus of the hydrogel is at most 50 MPa, more preferably at most 20 MPa, even more preferably at most 10 MPa and most preferably at most 5 MPa. Preferably the hydrogel has an elastic modulus between 0.1 and 10 MPa, more preferably between 0.3 and 5 MPa.

The hydrogel according to the invention can be used in a prosthetic nucleus pulposus. It has been found that using the hydrogel according to the invention can yield a prosthesis matching the behavior of the native nucleus in the joint as close as possible.

The advantage of the hydrogel according to the invention when used in the prosthetic nucleus pulposus is that the hydrogel is intrinsically radiopaque. An additional advantage of the prosthetic nucleus pulposus according to the invention is that the prosthesis can fill the entire cavity that is left after (complete) removal of the nucleus, thereby minimizing implant migration. A further advantage is that the prosthesis can be implanted in dehydrated form, which is relatively small, and is then allowed to hydrate and swell in situ. In dehydrated form the prosthesis can be between 1 and 5 times as small as in the swollen state.

It is possible to determine the swelling ratio of the prosthesis by measuring the swelling ratio of the hydrogel used therein. A method for measurement of the swelling ratio is given in the experimental part. Swelling ratios can be used to calculate the size of the nucleus prosthesis before implantation, based on the dimensions of the nucleus cavity. The person skilled in the art can thereby optimize the dimensions of the prosthesis before implantation in order to obtain a prosthesis, which will fill up the cavity completely after swelling to its equilibrium.

The prosthetic nucleus pulposus according to the invention can also comprise other components, for example anti-infectives and medicaments to improve healing of the annulus fibrosus after insertion of the prosthesis.

Furthermore, the invention relates to a method for forming a prosthetic nucleus pulposus comprising the steps of shaping a mass of bonded radiopaque hydrogel material as described above to a shape generally conforming to a natural human disc nucleus.

In one embodiment of the invention, the prosthetic nucleus pulposus according to the invention is formed before implantation. It can be implanted in swollen or in (partly) dehydrated form. Preferably the prosthetic nucleus pulposus is implanted in dehydrated form, being relatively small in this form, and is then allowed to swell in situ. A non-limiting Example of this method is depicted in Figures 1, 2 and 3. Figure 1 shows the native spine, comprising the intervertebral

disc (1), wherein the native nucleus pulposus (2) is surrounded by the annulus fibrosus (3) as located in the vertebra (4). In Figure 2, the native pulposus is removed and replaced by a dehydrated prosthetic nucleus pulposus (5), which in Figure 3 is swollen (5) to fill the entire cavity between the end plates and the annulus.

In another embodiment of the invention the prosthetic nucleus pulposus is formed and shaped in situ. This method comprising forming a mass of radiopaque hydrogel by injecting a mixture of at least

(a) a monomer comprising covalently bound iodine or bromine
(b) at least one hydrophilic monomer and
(c) optionally a hydrophobic monomer,

into the annulus in an amount such that upon expansion of the resulting hydrogel the annulus space is sufficiently filled to mimic a healthy nucleus.

The advantage of this method is that there is no need to make a large opening for insertion of the prosthesis. The natural nucleus generally can be removed by suction through a syringe, where after the mixture as described above can be injected.

The invention is hereafter illustrated by the following non-limiting Examples and comparative experiment.

Examples 1,2, 3, 4 and comparative experiment A

Materials and methods

Preparation of the copolymers

NVP and HEMA were purchased from Acros (Landsmeer, The Netherlands) and were distilled under reduced pressure to remove inhibiting additives. The monomer 4IEMA was synthesized from 4-iodobenzoyl chloride and purified HEMA. Purity and identity of all monomers was checked by $^1$H-NMR spectroscopy. 2,2'-azobis(isobutyronitrile) (AIBN) was used as the source of free radicals; this compound was used as received from Acros. Monomers and 0.044 mol% (based on the total amount of monomers) of AIBN were mixed and transferred to Teflon tubes, which were then immersed in a thermostated oil bath. The temperature profile shown in Table 1 was run.

*Table 1*: Temperature profile

| t (h) | 0 | 0.5 | 8.5 | 9.5 | 13.5 | 14.5 | 18.5 | 22.5 |
|---|---|---|---|---|---|---|---|---|
| T (°C) | 40 | 60 | 60 | 80 | 80 | 100 | 100 | 40 |

This resulted in transparent, glassy rods with a diameter of 12 mm. The compositions of the prepared copolymers are displayed in Table 2.

*Table 2*: Molar ratios of hydrogel composition

| Example | NVP | HEMA | 4IEMA | MMA |
|---|---|---|---|---|
| 1 | 95 | - | 5 | - |
| 2 | 90 | - | 10 | - |
| 3 | - | 95 | 5 | - |
| 4 | - | 90 | 10 | - |
| 5 | 94 | - | 6 | - |
| A | - | 19 | 21 | 60 |

Measurement of the elastic modulus (Young's modulus)

For testing the mechanical properties of the materials in a static experiment in compression, fully swollen cylindrical samples of ±8 mm thickness and ±15 mm diameter were used. Three samples of each material were compressed, both at room (20°C) and body temperature (37 °C), submerged in water, on a Zwick 1445 compression tester using a 500 N load cell at a strain rate of $3 \cdot 10^{-3}$ $s^{-1}$. The Young's modulus is calculated within the region of elastic deformation (±2% - 8% compression).

Measurement of Equilibrium Water Content

To study the water uptake of the different materials, swelling experiments in phosphate buffered saline (PBS) were conducted in triplet at room temperature (20°C) using sample discs (cut out of the glassy rods) with a diameter of 12 mm and a thickness of 2 mm. To assure a constant composition of the copolymers during swelling, all samples were washed and air-dried before the experiment. Material masses of the discs were measured at different time intervals. The sample was considered in the equilibrium state once the mass of the wetted sample did not change significantly (± 2%) within a week.

The excess of water from the surface was removed with a tissue before the samples were weighed. After weighing, the samples were put back in fresh, ample PBS. The equilibrium water content (EWC) was calculated using the following equation:

$$EWC = 100\% \times \frac{m_s - m_d}{m_s}$$

$m_s$ is the mass of the swollen sample in equilibrium state and $m_d$ is the mass of the dry sample.

Measurement of Swelling Ratio

In the same way as determining the equilibrium water content, the swelling ratio was determined, which is defined by the increase in volume: the volume of the swollen sample in equilibrium state, divided by the volume of the dry sample.

Radiopacity

X-ray visibility was checked by implanting swollen samples of Examples 3 and 4 into a nucleus cavity of the lumbar spine of a porcine cadaver by a certified orthopedic surgeon; subsequently X-ray photographs were taken of the materials in situ under standard hospital conditions, using a Philips BV Pulsera at 66kV and with automatic exposure.

Results

The results of the measurements of elastic modulus, swelling ratio and equilibrium water content (EWC) are shown in Table 3 below.

*Table 3* Results

| Example | Elastic modulus at 20°C (MPa) | Elastic modulus at 37°C (MPa) | Equilibrium water content (%) | Swelling ratio |
|---|---|---|---|---|
| 1 | 0.9 | 0.5 | 77.9 | 4.5 |
| 2 | 18 | 8 | 58.7 | 2.9 |
| 3 | 0.9 | 0.7 | 22.5 | 1.6 |
| 4 | 4 | 1 | 15.4 | 1.6 |
| 5 | 1 | 0.7 | 74 | 4 |
| A | 1200 | not determined | 2.8 | Not determined |

The NVP-based hydrogels of Examples 1, 2, and 5 have a higher equilibrium water content than the HEMA-based hydrogels. It also is shown that the presence of the hydrophobic 4IEMA decreases the water uptake of the hydrogels. Also the rate at which the hydrogels imbibe water depends on the hydrophilic character of the hydrogel. Whereas for the more hydrophilic hydrogels (1, 2, 5), equilibrium water content (EWC) is reached in about 12 hours, the less hydrophilic hydrogels (3, 4) take 2 days or more to reach equilibrium swelling.

Comparative experiment A clearly shows that the incorporation of the hydrophobic monomer MMA decreases the EWC and increases the elastic modulus too much for the material to be regarded as a hydrogel and is therefore not suitable for the purpose of the invention.

Radiopacity

Figure 4 shows the resulting X-ray image of the implantation whereby samples of Examples 3 (H95) and 4 (H90) were implanted between two adjacent vertebrae of a porcine cadaver. Three intervertebral structures are seen: the native disc (*) on the bottom, the sample of Example 4 in the middle and the sample of Example 3 on top. The native discus is seen as a non-absorbing (white) band, while samples of Experiments 3 and 4 are clearly visible as X-ray absorbing bands.

Figures 5 and 6 show respectively a CT-scan and an MRI scan of the sample of Example 5 implanted in a porcine cadaver. The CT-scan was made on a Toshiba Aquilion (at 120 kV, 20 mAs), and the MRI-scan was made on a Philips Gyroscan NT Intera 1.5T. The implant is clearly visible in a kidney-shaped form, completely filling the cavity.

**Claims**

**1.** Hydrogel suitable for a prosthetic nucleus pulposus, comprising a copolymer based on

(a) at least one monomer comprising covalently bound iodine or bromine, and;
(b) at least one hydrophilic monomer,

wherein the hydrogel, when fully hydrated, has an elastic modulus between 0.01-100 MPa at 37°C.

**2.** Hydrogel according to claim 1, wherein monomer (b) is chosen from the group consisting of N-vinyl-2-pyrrolidinone (NVP), 2-hydroxy ethyl methacrylate (HEMA), methacrylic acid (MAA), polyethylene glycol methacrylate (PEG-MA), vinyl acetate as a precursor for vinyl alcohol (VA) or derivatives thereof.

**3.** Hydrogel according to any one of claims 1-2, wherein the copolymer additionally comprises

(c) a hydrophobic monomer.

**4.** Hydrogel according to any one of claims 1-3, wherein the hydrogel at 20°C has an equilibrium water uptake between 10-95 wt% with respect to the wet weight of the hydrogel.

**5.** Prosthetic nucleus pulposus comprising a hydrogel according to any one of claims 1-4.

**6.** Method for forming a prosthetic nucleus pulposus comprising the steps of forming a mass of bonded radiopaque hydrogel material comprising a copolymer based on

(a) at least one monomer comprising covalently bound iodine or bromine
(b) at least one hydrophilic monomer

to a shape generally conforming to a natural disc nucleus.

**Patentansprüche**

**1.** Hydrogel, das für einen prothetischen Nucleus pulposus geeignet ist, umfassend ein Copolymer auf der Basis von

(a) wenigstens einem Monomer, umfassend kovalent gebundenes Iod oder Brom, und
(b) wenigstens einem hydrophilen Monomer,

wobei das Hydrogel, wenn vollständig hydratisiert, einen Elastizitätsmodul zwischen 0,01 und 100 MPa bei 37 °C aufweist.

**2.** Hydrogel gemäß Anspruch 1, wobei das Monomer (b) ausgewählt ist aus der Gruppe, bestehend aus N-Vinyl-2-pyrrolidinon (NVP), 2-Hydroxyethylmethacrylat (HEMA), Methacrylsäure (MAA), Polyethylenglycolmethacrylat (PEG-MA), Vinylacetat als Vorläufer für Vinylalkohol (VA) und Derivaten davon.

**3.** Hydrogel gemäß einem der Ansprüche 1-2, wobei das Copolymer zusätzlich

(c) ein hydrophobes Monomer

umfasst.

4. Hydrogel gemäß einem der Ansprüche 1-3, wobei das Hydrogel bei 20 °C eine Gleichgewichts-Wasseraufnahme zwischen 10 und 95 Gew.-%, bezogen auf das Feuchtgewicht des Hydrogels, aufweist.

5. Prothetischer Nucleus pulposus, umfassend ein Hydrogel gemäß einem der Ansprüche 1-4.

6. Verfahren zum Herstellen eines prothetischen Nucleus pulposus, umfassend die Schritte des Formens einer Masse von gebundenem röntgendichtem Hydrogelmaterial, umfassend ein Copolymer auf der Basis von

(a) wenigstens einem Monomer, umfassend kovalent gebundenes Iod oder Brom,
(b) wenigstens einem hydrophilen Monomer,

zu einer Form, die allgemein einem natürlichen Bandscheibenkern entspricht.

## Revendications

1. Hydrogel convenant pour la formation d'un noyau gélatineux prosthétique, comprenant un copolymère à base de

(a) au moins un monomère comprenant des atomes d'iode ou de brome liés par des liaisons covalentes, et ;
(b) au moins un monomère hydrophile,

l'hydrogel, quand il est entièrement hydraté, ayant un module élastique de 0,01 MPa à 100 MPa à 37 °C.

2. Hydrogel selon la revendication 1, dans lequel le monomère (b) est choisi dans le groupe constitué de la N-vinyl-2-pyrrolidinone (NVP), du méthacrylate de 2-hydroxyéthyle (HEMA), de l'acide méthacrylique (MAA), du méthacrylate de polyéthylène glycol (PEG-MA), de l'acétate de vinyle en tant que précurseur pour l'alcool vinylique (VA), ou de dérivés de ceux-ci.

3. Hydrogel selon l'une quelconque des revendications 1-2, dans lequel le copolymère comprend en outre (c)un monomère hydrophobe.

4. Hydrogel selon l'une quelconque des revendications 1-3, l'hydrogel à 20°C présentant une rétention d'eau d'équilibre de 10 % à 95 % en poids relativement au poids net de l'hydrogel.

5. Noyau gélatineux prosthétique comprenant un hydrogel selon l'une quelconque des revendications 1-4.

6. Procédé de formation d'un noyau gélatineux prosthétique, comprenant les étapes de formation d'une masse de matériau hydrogel radio-opaque aggloméré comprenant un copolymère à base de

(a) au moins un monomère comprenant des atomes d'iode ou de brome liés par des liaisons covalentes, et ;
(b) au moins un monomère hydrophile,

pour obtenir une forme généralement conforme à celle d'un noyau gélatineux naturel.

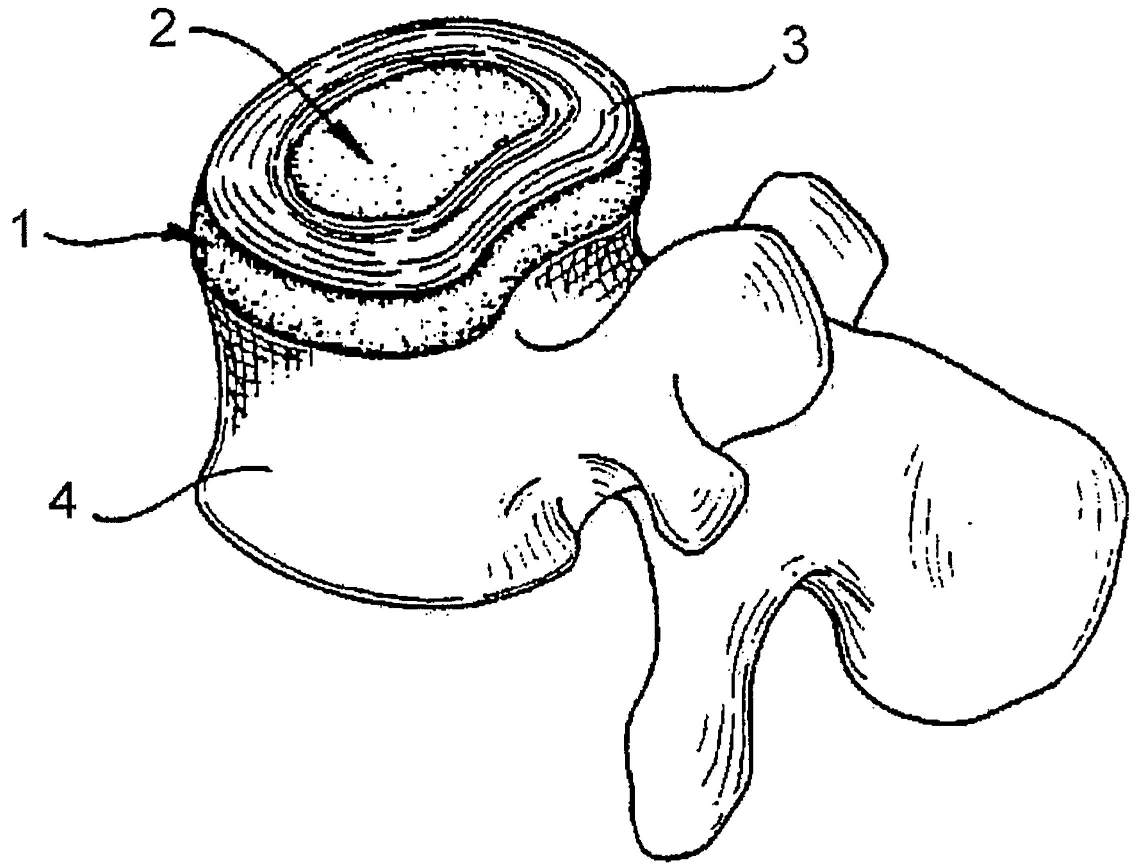
2
3
1
4

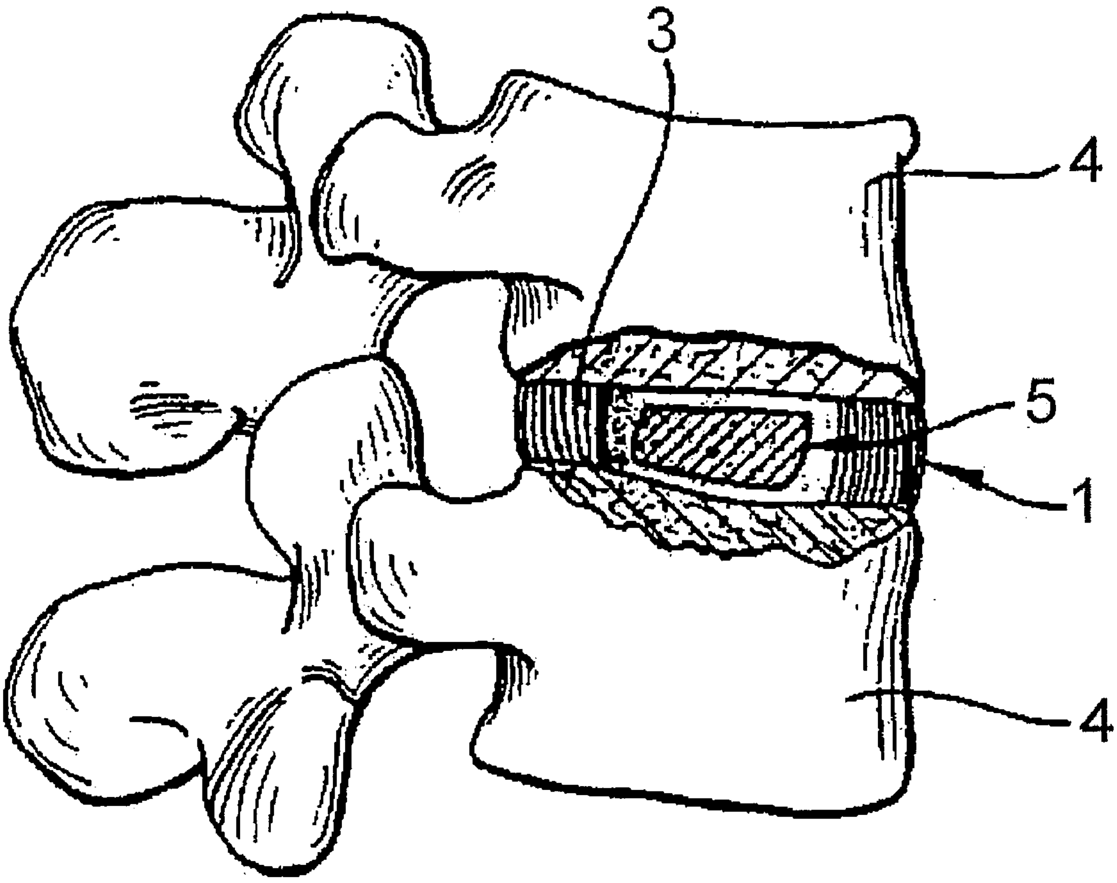
3
4
5
1
4

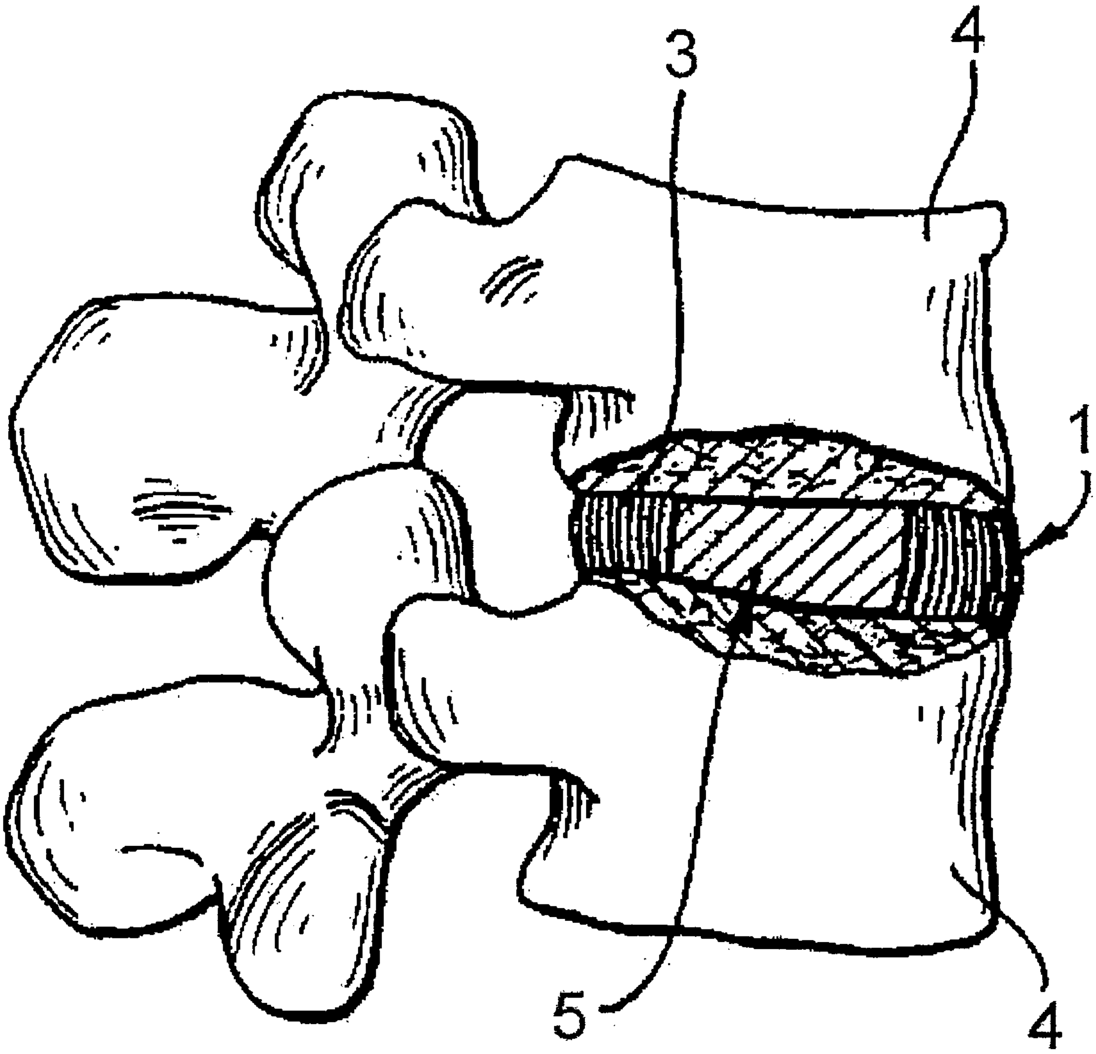
3
4
1
5
4

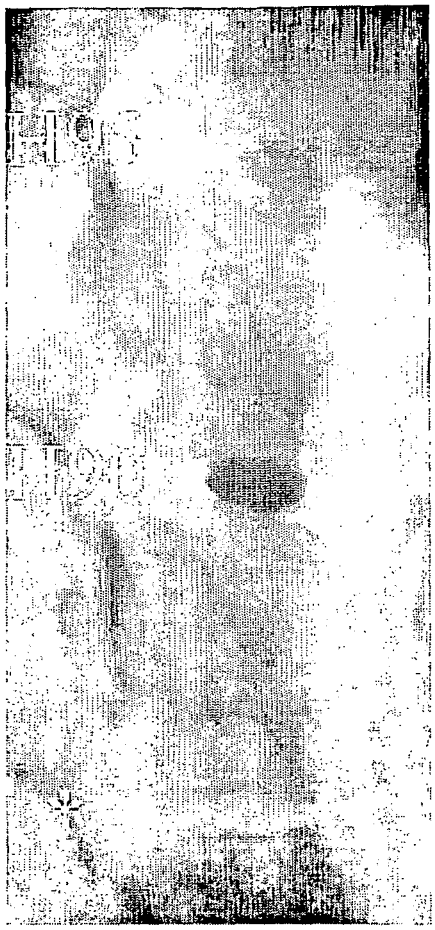

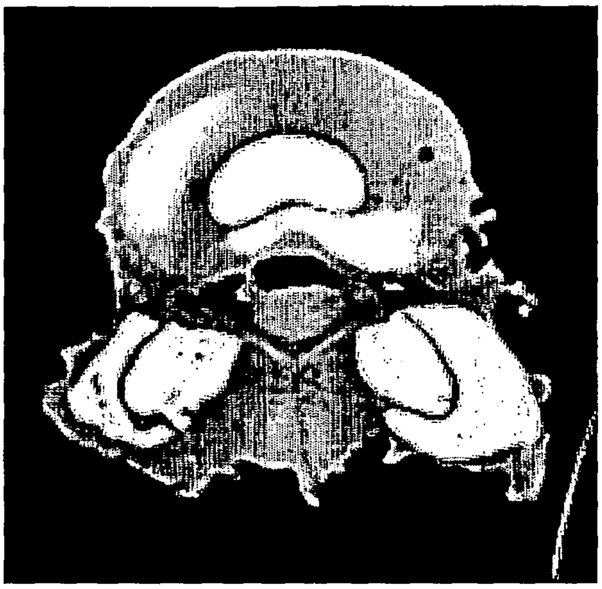

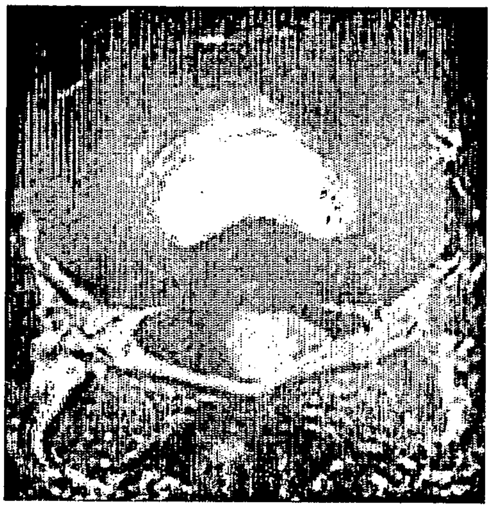

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9423671 A
- US 20040054413 A1
- WO 9605872 A


### Non-patent literature cited in the description


- **Peppas NA et al.** Hydrogels in pharmaceutical formulations. *Eur J Pharm Biopharm,* 2000, 27-46